# EUROPEAN PATENT APPLICATION

(11) **EP 3 848 029 A1**
(43) Date of publication of application: **14.07.2021**
(21) Application number: 19845227.8
(22) Date of filing: 30.07.2019
(51) Int. Cl.: A61K 31/4418, A61K 38/49, A61P 7/02, A61P 9/10, A61P 43/00

(54) **MEDICINE COMPOSITION FOR TREATING CEREBRAL INFARCTION**

(30) Priority: 31.07.2018 JP 2018143437
(71) Applicant: Kyoto University, Kyoto-shi, Kyoto 606-8501 (JP)
(72) Inventor: KAKIZUKA, Akira, Kyoto-shi, Kyoto 606-8501 (JP); KINOSHITA, Hisanori, Kyoto-shi, Kyoto 606-8501 (JP); MAKI, Takakuni, Kyoto-shi, Kyoto 606-8501 (JP); TAKAHASHI, Ryosuke, Kyoto-shi, Kyoto 606-8501 (JP)
(74) Representative: Hoffmann Eitle
(86) International application number: PCT/JP2019/029844
(87) International publication number: WO 2020/027137

(57) **Abstract**

The disclosure provides a compound of formula (I) for treating cerebral infarction, a pharmaceutical composition comprising the compound, a method of manufacturing a pharmaceutical composition for treating cerebral infarction comprising use of the compound, use of the compound for manufacturing a pharmaceutical composition for treating cerebral infarction, or a method for treating cerebral infarction comprising administering the compound or the pharmaceutical composition. The cerebral infarction includes, for example, lacunar infarction, atherothrombotic cerebral infarction, and cardiogenic embolism.

## Description

### TECHNICAL FIELD

This application claims the benefit of priority of Japanese Patent Application No. 2018-143437, the entire contents of which are incorporated herein by reference.

The disclosure relates to a pharmaceutical composition for treating cerebral infarction.

### BACKGROUND

Cerebral infarction is characterized in that cerebral ischemia is caused by occlusion or stenosis of a cerebral artery and a brain tissue necrotizes completely or partially due to insufficient oxygen or nutrients supply. Cerebral infarction includes lacunar infarction, which is caused by a lesion in an intracerebral small artery; atherothrombotic cerebral infarction, which is caused by atherosclerosis of a relatively large artery in the cervical or cranial region; and cardiogenic embolism, which is caused by a cardiac disease. Cerebral infarction is a major issue in terms of patient QOL, welfare, and medical economy, since the mortality rate due to cerebral infarction is high, and once it occurs, patients are highly likely to suffer from aftereffects.

In the acute phase of cerebral infarction, thrombolytic therapy using tissue plasminogen activator (tPA or t-PA) is the first-line choice. Plasminogen is an enzyme that strongly dissolves thrombi formed in blood vessels. After cerebral infarction is caused by a thrombus in a brain artery, tPA is administered to dissolve the thrombus and make the blood flow reperfuse (thrombolytic therapy). It has been reported that 40 to 50% of the patients treated with tPA recovers from the disease and becomes capable of living independently. However, tPA can be administered only within 4.5 hours from the onset of an acute stroke, because tPA may cause cerebral hemorrhage when administered after a long time from the onset. Recently, an intravascular therapy which removes a thrombus from a blood vessel by mechanical thrombectomy has been used for treatable patients within eight hours from the onset. Nevertheless, further enhancement of the recovery rate is strongly desired in clinical practice and developing a novel therapy for cerebral infarction is a very important issue.

Certain 4-amino-naphthalene-1-sulfonic acid derivatives have a VCP (valosin-containing protein) ATPase inhibitory activity and are considered to be effective for treating various diseases (Patent Literature 1). Especially, they are known to be effective in treatment or prevention of some ocular diseases and leptin resistance (Patent Literatures 2 to 5).

### REFERENCES

### PATENT LITERATURE

[Patent Literature 1] WO2012/014994
[Patent Literature 2] WO2012/043891
[Patent Literature 3] WO2014/129495
[Patent Literature 4] WO2015/129809
[Patent Literature 5] WO2015/033981

### SUMMARY

An object of the disclosure is to provide a pharmaceutical composition for treating cerebral infarction.

The inventors have found that VCP modulators can protect cortical neurons and are effective for treating cerebral infarction.

Accordingly, an aspect of the disclosure provides a pharmaceutical composition for treating cerebral infarction comprising a compound of formula (I): 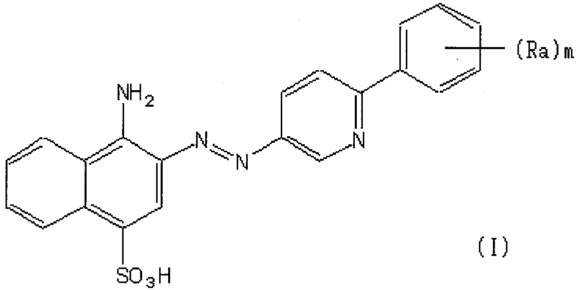 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt or solvate thereof.

According to the disclosure, the pharmaceutical composition for treating cerebral infarction is provided.

### BRIEF DESCRIPTION OF DRAWINGS

Fig. 1 illustrates procedures for *in vitro* experiments using primary cortical neurons.
Fig. 2 shows the neuronal viability after oxygen-glucose deprivation (OGD) in the presence and the absence of KUS121.
Fig. 3 shows the MAP2-positive area in neurons after OGD in the presence and the absence of KUS121.
Fig. 4 shows the ATP level in neurons after OGD in the presence and the absence of KUS121.
Fig. 5 shows the expression of C/EBP-homologous protein (CHOP) in neurons after tunicamycin treatment in the presence and the absence of KUS121.
Fig. 6 illustrates procedures for *in vivo* experiments using mice.
Fig. 7 shows the rotarod retention time of C57BL/6 mice which were treated with KUS121 or vehicle after induction of transient focal cerebral ischemia.
Fig. 8 shows the infarction volumes in brains of C57BL/6 mice which were treated with KUS121 or vehicle after induction of transient focal cerebral ischemia.
Fig. 9 shows the infarction volumes in brains of CB-17 mice which were treated with KUS121 or vehicle before induction of transient focal cerebral ischemia.
Fig. 10 shows the results of Western blotting indicating the expression of NeuN in cerebral cortex of CB-17 mice which were treated with KUS121 or vehicle before induction of transient focal cerebral ischemia.

### DETAILED DESCRIPTION

When a numerical value is accompanied with the term "about", the value is intended to represent any value in the range of -10% of the value to +10% of the value. For example, "about 20" means "a value from 18 to 22." A range defined with values of the lower and upper limits covers all values from the lower limit to the upper limit, including the values of the both limits. When a range is accompanied with the term "about", the both limits are read as accompanied with the term. For example, "about 20 to 30" is read as "18 to 33."

Unless otherwise defined, the terms used herein are read as generally understood by those skilled in the technical fields such as organic chemistry, medical sciences, pharmaceutical sciences, molecular biology, and microbiology. Several terms used herein are defined as below. The definitions herein take precedence over the general understanding.

The term "alkyl" refers to a monovalent saturated aliphatic hydrocarbyl group having 1 to 10, preferably 1 to 6, carbon atoms. Examples of the alkyl groups include, but are not limited to, linear and branched hydrocarbyl groups, such as methyl, ethyl, n-propyl, isopropyl, n-butyl, isobutyl, sec-butyl, t-butyl, n-pentyl, and neopentyl.

The term "substituted" as a word qualifying a name of a group indicates that one or more hydrogen atoms of the group are, identically or differently, replaced with one or more designated substituents.

The term "alkylene" refers to a divalent saturated aliphatic hydrocarbyl group having 1 to 10, preferably 1 to 6, carbon atoms. Alkylene groups include branched and straight chain hydrocarbyl groups.

The term "alkoxy" refers to an -O-alkyl group in which the alkyl group is as defined herein. Examples of the alkoxy groups include, but are not limited to, methoxy, ethoxy, n-propoxy, isopropoxy, n-butoxy, t-butoxy, sec-butoxy, and n-pentoxy.

The term "aryl" refers to a monovalent aromatic carbocyclic group of 6 to 14 carbon atoms having a single ring (e.g., phenyl) or multiple condensed rings (e.g., naphthyl or anthryl). Typical aryl groups include phenyl and naphthyl.

The term "aryloxy" refers to an -O-aryl group in which the aryl group is as defined herein. Examples of the aryloxy groups include phenoxy and naphthoxy.

The term "cyano" refers to the -CN group.

The term "carboxyl" or "carboxy" refers to the -COOH group or a salt thereof.

The term "carboxy ester" refers to a -C(O)O-alkyl group in which the alkyl group is as defined herein.

The term "halo" refers to a halogen, especially fluoro, chloro, bromo, or iodo.

The term "hydroxy" refers to the -OH group.

A substituent that is not explicitly defined herein is named by describing the name of the terminal functional group of the substituent first and sequentially describing the adjacent functional group(s) toward the point binding to the rest of the compound, unless otherwise indicated. For example, the substituent "arylalkyloxycarbonyl" refers to (aryl)-(alkyl)-O-C(O)-.

Some compounds of formula (I) have enantiomers or diastereomers, depending on arrangements of their substituents. Some compounds of formula (I) may be provided as racemic mixtures or may be provided in stereoisomerically pure forms separated by a known method. Some compounds of formula (I) may be tautomers.

The term "ester" refers to an ester that hydrolyzes in vivo, which may break down readily in a human body to leave the parent compound or a salt thereof. Suitable esters include, for example, those derived from pharmaceutically acceptable aliphatic carboxylic acids, especially alkanoic, alkenoic, cycloalkanoic, and alkanedioic acids, in which each alkyl or alkenyl group has, for example, not more than six carbon atoms. Examples of the esters include formates, acetates, propionates, butyrates, acrylates, and ethylsuccinates.

The term "oxide" refers to an oxide in which a nitrogen in a heteroaryl group is oxidized to form N-oxide.

The term "pharmaceutically acceptable salt" may indicate a salt of a compound of formula (I) with an inorganic or organic acid. Preferred salts include salts with inorganic acids, such as hydrochloric acid, hydrobromic acid, phosphoric acid, and sulfuric acid, and salts with organic carboxylic acids and sulfonic acids, such as acetic acid, trifluoroacetic acid, propionic acid, maleic acid, fumaric acid, malic acid, citric acid, tartaric acid, lactic acid, benzoic acid, methanesulfonic acid, ethanesulfonic acid, benzenesulfonic acid, toluenesulfonic acid, naphthalene sulfonic acid, and naphthalene disulfonic acid.

Pharmaceutically acceptable salts also include salts with conventional bases, such as alkali metal salts, e.g., sodium and potassium salts, alkaline earth metal salts, e.g., calcium and magnesium salts, ammonium salts derived from ammonia and organic amines, e.g., diethylamine, triethylamine, ethyldiisopropylamine, procaine, dibenzylamine, N-methylmorpholine, dihydroabiethylamine, methylpiperidine, L-arginine, creatine, choline, L-lysine, ethylenediamine, benzathine, ethanolamine, meglumine, and tromethamine, especially a sodium salt.

The term "solvate"' means a compound of formula (I) which is coordinated with a solvent molecule in a solid or liquid state to form a complex. A suitable solvate is a hydrate.

The term "compound of formula (I)" as used herein is intended to include its esters, oxides, pharmaceutically acceptable salts, and solvates, as long as the context allows it.

In an embodiment, each Ra radical in formula (I) is independently selected from the group consisting of halo, hydroxy, alkyl, halo-substituted alkyl, and alkoxy.

In an embodiment, each Ra radical in formula (I) is independently selected from the group consisting of halo and alkyl.

In an embodiment, formula (I) has two Ra radicals which are halo and alkyl.

In an embodiment, the compound of formula (I) is selected from the compounds listed in Table 1 below:

**[Table 1-1]**

| No. | Compound Name |
|---|---|
| 1 | 4-amino-3-(6-phenylpyridine-3-ylazo)naphthalene-1-sulfonic acid |
| 2 | 4-amino-3-(6-p-tolylpyridine-3-ylazo)naphthalene-1-sulfoni c acid |
| 3 | 4-amino-3-(6-m-tolylpyridine-3-ylazo)naphthalene-1-sulfoni c acid |
| 4 | 4-amino-3-(6-o-tolylpyridine-3-ylazo)naphthalene-1-sulfoni c acid |
| 5 | 4-amino-3-(6-biphenyl-2-ylpyridine-3-ylazo)naphthalene-1-s ulfonic acid |
| 6 | 3-[6-(2-acetylphenyl)pyridine-3-ylazo]-4-aminonaphthalene-1-sulfonic acid |
| 7 | 3-[6-(3-acetylphenyl)pyridine-3-ylazo]-4-aminonaphthalene-1-sulfonic acid |
| 8 | 3-[6-(4-acetylphenyl)pyridine-3-ylazo]-4-aminonaphthalenes ulfonic acid |
| 9 | 4-amino-3-[6-(2,4-dichlorophenyl)pyridine-3-ylazo]naphthal ene-1-sulfonic acid |
| 10 | 4-amino-3-[6-(2-trifluoromethylphenyl)pyridine-3-ylazo]nap hthalene-1-sulfonic acid |
| 11 | 4-amino-3-[6-(4-trifluoromethylphenyl)pyridine-3-ylazo]nap hthalene-1-sulfonic acid |
| 12 | 4-amino-3-[6-(2-chlorophenyl)pyridine-3-ylazo]naphthalene-1-sulfonic acid |
| 13 | 4-amino-3-[6-(3-chlorophenyl)pyridine-3-ylazo]naphthalene-1-sulfonic acid |
| 14 | 4-amino-3-[6-(4-chlorophenyl)pyridine-3-ylazo]naphthalene-1-sulfonic acid |
| 15 | 4-amino-3-[6-(2-methoxyphenyl)pyridine-3-ylazo]naphthalene -1-sulfonic acid |
| 16 | 4-amino-3-[6-(4-methoxyphenyl)pyridine-3-ylazo]naphthalene -1-sulfonic acid |
| 17 | 4-amino-3-[6-(2-isopropoxyphenyl)pyridine-3-ylazo]naphthal ene-1-sulfonic acid |
| 18 | 4-amino-3-[6-(4-isopropoxyphenyl)pyridine-3-ylazo]naphthal ene-1-sulfonic acid |
| 19 | 4-amino-3-[6-(2-phenoxyphenyl)pyridine-3-ylazo]naphthalene -1-sulfonic acid |
| 20 | 4-amino-3-[6-(3-methoxyphenyl)pyridine-3-ylazo]naphthalene -1-sulfonic acid |

**[Table 1-2]**

| | |
|---|---|
| 21 | 4-amino-3-[6-(2,3-dimethylphenyl)pyridine-3-ylazo]naphthal ene-1-sulfonic acid |
| 22 | 4-amino-3-[6-(2,5-dimethylphenyl)pyridine-3-ylazo]naphthal ene-1-sulfonic acid |
| 23 | 4-amino-3-[6-(3,5-dimethylphenyl)pyridine-3-ylazo]naphthal ene-1-sulfonic acid |
| 24 | 4-amino-3-[6-(3-trifluoromethylphenyl)pyridine-3-ylazo]nap hthalene-1-sulfonic acid |
| 25 | 4-{4-[5-(1-amino-4-sulfonaphthalene-2-ylazo)pyridine-2-yl ]phenyl}-4-oxobutyric acid |
| 26 | 4-amino-3-(6-biphenyl-3-ylpyridine-3-ylazo)naphthalene-1-s ulfonic acid |
| 27 | 4-amino-3-[6-(3-cyanophenyl)pyridine-3-ylazo]naphthalene-1 -sulfonic acid |
| 28 | 4-amino-3-[6-(4-cyanophenyl)pyridine-3-ylazo]naphthalene-1 -sulfonic acid |
| 29 | 4-amino-3-[6-(3,5-bistrifluoromethylphenyl)pyridine-3-ylaz o]naphthalenesulfonic acid |
| 30 | 4-amino-3-[6-(4-benzoylphenyl)pyridine-3-ylazo]naphthalene -1-sulfonic acid |
| 31 | 4-amino-3- [6- (2-propoxyphenyl) pyridine-3-ylazo] naphthalen e-1-sulfonic acid |
| 32 | 4-amino-3-[6-(4-fluoro-2-methylphenyl)pyridine-3-ylazo]nap hthalene-1-sulfonic acid |
| 33 | 4-amino-3-[6-(5-fluoro-2-propoxyphenyl)pyridine-3⁻ylazo]na phthalene-1-sulfonic acid |
| 34 | 4-amino-3-[6-(2-fluoro-6-propoxyphenyl)pyridine-3-ylazo]na phthalene-1-sulfonic acid |
| 35 | 4-amino-3-[6-(4-fluoro-2-propoxyphenyl)pyridine-3-ylazo]na phthalene-1-sulfonic acid |
| 36 | 4-amino-3-[6-(5-fluoro-2-methylphenyl)pyridine-3-ylazo]nap hthalene-1-sulfonic acid |
| 37 | 4-amino-3-[6-(2-fluoro-5-methylphenyl)pyridine-3-ylazo]nap hthalene-1-sulfonic acid |
| 38 | 4-amino-3-[6-(2-butoxyphenyl)pyridine-3-ylazo]naphthalene-1-sulfonic acid |
| 39 | 4-amino-3-[6-(2-hexyloxyphenyl)pyridine-3-ylazo]naphthalen e-1-sulfonic acid |
| 40 | 4-amino-3-[6-(4-butylphenyl)pyridine-3-ylazo]naphthalene-1 -sulfonic acid |

**[Table 1-3]**

| | |
|---|---|
| 41 | 4-amino-3-[6-(2-hydroxyphenyl)pyridine-3-ylazo]naphthalene -1-sulfonic acid |
| 42 | 4-amino-3-{6-[2-(6-hydroxyhexyloxy)phenyl]pyridine-3-ylazo }naphthalene-1-sulfonic acid |
| 43 | 4-{2-[5-(1-amino-4-sulfonaphthalene-2-ylazo)pyridine-2-yl] phenoxy}butyric acid |
| 44 | 4-amino-3-{6-[2-(3-hydroxypropoxy)phenyl]pyridine-3-ylazo} naphthalene-1-sulfonic acid |
| 45 | 4-amino-3-[6-(2-isobutoxyphenyl)pyridine-3-ylazo]naphthale ne-1-sulfonic acid |
| 46 | 4-amino-3-[6-(5-chloro-2-hydroxyphenyl)pyridine-3-ylazo]na phthalene-1-sulfonic acid |
| 47 | 4-amino-3-[6-(4-methylbiphenyl-2-yl)pyridine-3-ylazo]napht halene-1-sulfonic acid |
| 48 | 4-amino-3-[6-(4'-chloro-4-methylbiphenyl-2-yl)pyridine-3-y lazo]naphthalene-1-sulfonic acid |
| 49 | 4-amino-3-[6-(4,3',5'-trimethylbiphenyl-2-yl)pyridine-3-yl azo]naphthalene-1-sulfonic acid |
| 50 | 4-amino-3-[6-(3'-chloro-4-methylbiphenyl-2-yl)pyridine-3-y lazo]naphthalene-1-sulfonic acid |
| 51 | 4-amino-3-[6-(2,6-dimethylphenyl)pyridine-3-ylazo]naphthal ene-1-sulfonic acid |
| 52 | 4-amino-3-[6-(3-formyl-2-isopropoxy-5-methylphenyl)pyridin e-3-ylazo]naphthalene-1-sulfonic acid |
| 53 | 4-amino-3-[6-(3-formyl-2-butoxy-5-methylphenyl)pyridine-3-ylazo]naphthalene-1-sulfonic acid |

In an embodiment, the active ingredient of the pharmaceutical composition is 4-amino-3-[6-(4-fluoro-2-methylphenyl)pyridine-3-ylazo]naphthalene-1-sulfonic acid, which is represented by the following formula: 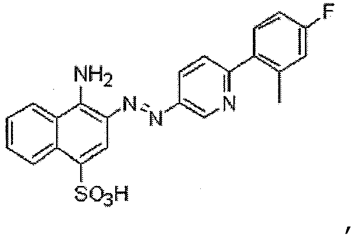 or an ester, oxide, pharmaceutically acceptable salt or solvate thereof, preferably the sodium salt.

The characteristics of the compounds of formula (I), especially the compounds listed above, and the methods for synthesizing them are described in WO2012/014994(Patent Literature 1) in detail.

The term "cerebral infarction" as used herein means a damage in the brain caused by cerebral ischemia, including, but not being limited to, lacunar infarction, atherothrombotic cerebral infarction, and cardiogenic embolism. The cause of cerebral ischemia includes, but is not limited to, cerebral thrombosis, cerebral embolism, vasospasm, hypotension, and hypoxemia. The term "cerebral infarction" as used herein includes symptoms related to cerebral infarction and sequelae of cerebral infarction, such as neuropathy, higher brain dysfunction, and affective disorder.

The term "treatment" as used herein encompasses any medical intervention intended to cure, ameliorate, palliate, alleviate and/or temporarily ameliorate a disease or condition. For example, "treatment" encompasses medical interventions for a variety of purposes, including delay or stop of disease progression, regression or disappearance of a lesion, or prevention of recurrence.

The subjects of the treatment include animals, typically mammals (e.g., humans, mice, rats, hamsters, rabbits, cats, dogs, cows, sheep, or monkeys), especially humans.

The pharmaceutical composition may be administered in any way through a conventional administration route, for example, by oral administration, parenteral administration, injection, or infusion. The composition may be in a dosage form suitable for each administration route. The composition may also be administered intrathecally, extradurally, or intraventricularly. In an embodiment, the pharmaceutical composition is administered intravenously.

Dosage forms suitable for oral administration include granules, fine granules, powders, coated tablets, tablets, suppositories, fine powders, capsules, microcapsules, chewable tablets, liquids, suspensions, and emulsions. Dosage forms suitable for injection may be conventional dosage forms, e.g., those suitable for intravenous injection, infusion, or formulations for extended release of active ingredients. Dosage forms for intravenous injection or infusion include aqueous and non-aqueous injectable solutions, which may comprise excipients, such as antioxidants, buffers, bacteriostatic agents, or isotonic agents; and aqueous and non-aqueous injectable suspensions, which may comprise excipients, such as suspending agents or thickening agents. Such dosage forms may be provided as liquids in sealed ampoules or vials, or provided as lyophilized products and prepared immediately prior to use by adding sterile liquids such as water for injection. The injectable solutions or suspensions may be prepared from powders, granules, or tablets.

Such dosage forms can be manufactured by formulating an active ingredient by a conventional method. If necessary for the formulation, any one of various pharmaceutically acceptable excipients may be added. Any excipient may be used in accordance with the employed dosage form. Examples of the excipients include buffering agents, surfactants, stabilizers, preservatives, fillers, diluents, additives, disintegrants, binders, coating agents, lubricants, lubricating agents, flavoring agents, sweeteners, and solubilizers.

The dosage and the number of doses of the pharmaceutical composition may be appropriately set by those skilled in the art so that an effective amount of a compound of formula (I) is administered to the subject, on the basis of factors such as the animal species, health condition, age, and weight of the subject, the administration route, and the employed dosage form. Those skilled in the art may easily determine the effective amount in a given situation by routine experimentation, which is within the range of ordinary skill and determination of clinicians. For example, a compound of formula (I) may be administered in the range of about 0.001 to 1000 mg/kg body weight/day, about 0.01 to 300 mg/kg body weight/day, or about 0.1 to 100 mg/kg body weight/day. For example, the pharmaceutical composition may be administered in a single dose or in multiple doses, or may be continuously administered.

In an embodiment, the pharmaceutical composition is administered during the acute phase of cerebral infarction. The term "acute phase" means a period of 14 days from the onset of cerebral infarction. The pharmaceutical composition may be administered to a subject after the onset of cerebral infarction, for example, immediately after the onset of cerebral infarction, e.g., within about 3, about 4.5, about 6, about 8, about 12, or about 24 hours after the onset of cerebral infarction. In the disclosure, the time at which the cause of cerebral ischemia (e.g., occlusion or stenosis of a cerebral artery) occurs is considered to be the time of the onset of cerebral infarction. In an embodiment, the pharmaceutical composition is administered in a single dose or in multiple doses during the acute phase of cerebral infarction. In an embodiment, the pharmaceutical composition is administered continuously throughout or during a part of the acute phase of cerebral infarction. In an embodiment, the pharmaceutical composition is administered before the inflammation due to cerebral infarction occurs. The presence or absence of inflammation can be determined on the basis of an inflammatory marker such as CRP or IL-1β in the blood.

In an embodiment, the pharmaceutical composition may be administered immediately before or immediately after a reperfusion procedure of the occluded vessel, e.g., by administration of a thrombolytic agent or by surgery. The term "immediately before a reperfusion procedure" means a time point within a period from several minutes or several tens of minutes before the reperfusion procedure, e.g., about 60 minutes, about 30 minutes, about 20 minutes, about 15 minutes, about 10 minutes, about 5 minutes, or about 3 minutes before the reperfusion procedure, to the reperfusion procedure. The term "immediately after a reperfusion procedure" means a time point within a period from the reperfusion procedure to several minutes or several tens of minutes after the reperfusion procedure, e.g., about 60 minutes, about 30 minutes, about 20 minutes, about 15 minutes, about 10 minutes, about 5 minutes, or about 3 minutes after the reperfusion procedure. In an embodiment, the pharmaceutical composition is administered simultaneously with the reperfusion procedure. Alternatively, the reperfusion procedure may be performed while the pharmaceutical composition is being administered continuously.

In an embodiment, about 1 to 1000 mg/kg body weight, about 5 to 500 mg/kg body weight, about 10 to 300 mg/kg body weight, or about 25 to 200 mg/kg body weight, e.g. about 100 mg/kg body weight, of a compound of formula (I) may be intravenously administered in a single dose during the acute phase.

A compound of formula (I) may be used alone or in combination with at least one further active ingredient, especially an active ingredient for treating cerebral infarction. Examples of the active ingredients suitable for use in combination include, but are not limited to, thrombolytic agents such as tPA, rtPA (e.g., alteplase, monteplase, pamiteplase, desmoteplase, reteplase, tenecteplase), and urokinase, antiplatelet agents (e.g., ozagrel sodium, aspirin, clopidogrel, and cilostazol), selective thrombin inhibitors (e.g., argatroban), heparin, low molecular weight heparin, heparinoid, cerebroprotective agents (e.g., edaravone), hypertonic glycerol, hypertonic mannitol, vasodilator agents, anti-inflammatory agents, and statins, especially rtPA, especially alteplase.

When some ingredients are used in combination, a dosage form containing all the ingredients or a combination of dosage forms containing the ingredients separately may be employed. The ingredients may be simultaneously administered or any ingredient may be administered at a later time point, as long as the ingredients are used for treating cerebral infarction. Two or more further active ingredients may be used in combination.

A non-drug therapy may be combined with the administration of a compound of formula (I). Examples of the suitable therapies include surgical procedures such as mechanical thrombectomy, decompressive craniectomy, emergency carotid endarterectomy, acute recanalization therapy, and acute carotid revascularization (angioplasty/stent implantation), gene therapy, and regenerative therapy.

An aspect of the disclosure provides a method for treating cerebral infarction comprising administering an effective amount of a compound of formula (I) to a subject in need thereof.

An aspect of the disclosure provides a compound of formula (I) for use in treating cerebral infarction.

An aspect of the disclosure provides use of a compound of formula (I) for treating cerebral infarction.

An aspect of the disclosure provides use of a compound of formula (I) for manufacturing a pharmaceutical composition for treating cerebral infarction.

For example, the disclosure provides the following embodiments.
[1] A pharmaceutical composition for treating cerebral infarction, comprising a compound of formula (I): 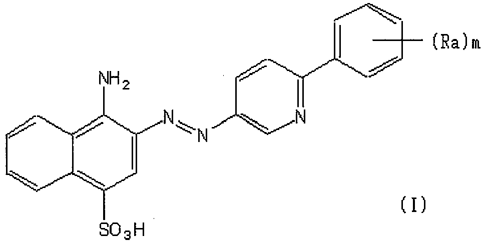 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt or solvate thereof.
[2] The pharmaceutical composition according to item 1, wherein each Ra radical is independently selected from the group consisting of halo, hydroxy, alkyl, halo-substituted alkyl, and alkoxy.
[3] The pharmaceutical composition according to item 1 or 2, wherein each Ra radical is independently selected from the group consisting of halo and alkyl.
[4] The pharmaceutical composition according to any one of items 1 to 3, wherein formula (I) has two Ra radicals which are halo and alkyl.
[5] The pharmaceutical composition according to any one of items 1 to 4, wherein the compound of formula (I) is selected from the compounds listed in Table 1.
[6] The pharmaceutical composition according to any one of items 1 to 5, wherein the compound of formula (I) is 4-amino-3-[6-(4-fluoro-2-methylphenyl)pyridine-3-ylazo]naphthalene-1-sulfonic acid.
[7] The pharmaceutical composition according to any one of items 1 to 6, which comprises 4-amino-3-[6-(4-fluoro-2-methylphenyl)pyridine-3-ylazo]naphthalene-1-sulfonic acid sodium salt.
[8] The pharmaceutical composition according to any one of items 1 to 7, wherein the cerebral infarction is lacunar infarction, atherothrombotic cerebral infarction, or cardiogenic embolism.
[9] The pharmaceutical composition according to any one of items 1 to 8, wherein the cerebral infarction is lacunar infarction.
[10] The pharmaceutical composition according to any one of items 1 to 9, wherein the composition is administered during the acute phase of the cerebral infarction.
[11] The pharmaceutical composition according to any one of items 1 to 10, wherein the composition is administered immediately before or immediately after a reperfusion procedure after the cerebral infarction.
[12] The pharmaceutical composition according to any one of items 1 to 10, wherein the composition is administered simultaneously with a reperfusion procedure after the cerebral infarction.
[13] The pharmaceutical composition according to item 11 or 12, wherein the reperfusion procedure is administration of a thrombolytic agent.
[14] The pharmaceutical composition according to item 11 or 12, wherein the reperfusion procedure is a surgical procedure.
[15] The pharmaceutical composition according to any one of items 1 to 14, wherein the composition is administered in a single dose.
[16] The pharmaceutical composition according to any one of items 1 to 14, wherein the composition is administered in multiple doses.
[17] The pharmaceutical composition according to any one of items 1 to 14, wherein the composition is continuously administered.
[18] The pharmaceutical composition according to any one of items 1 to 17, wherein the composition is administered within about 24 hours after the onset of the cerebral infarction.
[19] The pharmaceutical composition according to any one of items 1 to 18, wherein the composition is administered within about 12 hours after the onset of the cerebral infarction.
[20] The pharmaceutical composition according to any one of items 1 to 19, wherein the composition is administered within about 8 hours after the onset of the cerebral infarction.
[21] The pharmaceutical composition according to any one of items 1 to 20, wherein the composition is administered within about 6 hours after the onset of the cerebral infarction.
[22] The pharmaceutical composition according to any one of items 1 to 21, wherein the composition is administered within about 4.5 hours after the onset of the cerebral infarction.
[23] The pharmaceutical composition according to any one of items 1 to 22, wherein the composition is administered within about 3 hours after the onset of the cerebral infarction.
[24] The pharmaceutical composition according to any one of items 1 to 23, wherein about 1 to 1000 mg/kg body weight of the compound of formula (I) is administered.
[25] The pharmaceutical composition according to any one of items 1 to 24, wherein about 5 to 500 mg/kg body weight of the compound of formula (I) is administered.
[26] The pharmaceutical composition according to any one of items 1 to 25, wherein about 10 to 300 mg/kg body weight of the compound of formula (I) is administered.
[27] The -pharmaceutical composition according to any one of items 1 to 26, wherein about 25 to 200 mg/kg body weight of the compound of formula (I) is administered.
[28] The pharmaceutical composition according to any one of items 1 to 27, wherein the composition is used in combination with a thrombolytic agent.
[29] The pharmaceutical composition according to item 28, wherein the thrombolytic agent is tPA.
[30] The pharmaceutical composition according to item 28 or 29, wherein the thrombolytic agent is alteplase.
[31] The pharmaceutical composition according to any one of items 1 to 30, wherein the composition is intravenously administered.

The entire contents of the documents cited herein are incorporated herein by reference.

The embodiments described above are non-limiting and may be modified without deviating from the scope of the invention as defined by the appended claims. The following example is non-limiting and provided only for describing the invention.

### EXAMPLE 1

### Materials and methods

### Primary neuronal cell culture

Cortical neuronal cultures were prepared from 17-day-old Sprague Dawley rat embryos (Shimizu Laboratory Supplies) using methods described earlier (Maki T, et al, Annals of Clinical and Translational Neurology, 2014 Aug;1(8):519-33). Briefly, cortices were dissected and dissociated. Cells were plated on dishes coated with poly-D-lysine in Dulbecco's modified Eagle's medium (DMEM) containing 5% heat-inactivated fetal bovine serum and 1% penicillin/streptomycin at a density of 200, 000-250, 000 cells/cm². At 24-hour after seeding, the medium was changed to Neurobasal (NB) medium containing 0.5 mM glutamine, 1% penicillin/streptomycin and 2% B27 supplement. Cultured neurons were used for experiments 14 days after seeding.

### Oxygen glucose deprivation

The medium was replaced with DMEM with no glucose. Then, the cells were placed into the sealed Anaero container with an Anaero Pack (Mitsubishi Gas Chemical Company) for 1.5-2 hours. Following oxygen glucose deprivation, cells were switched to the normal medium and returned to a normoxic 5% CO₂ incubator.

### Cell viability test

Cell viability was assessed by Cell Counting Kit-8 (CCK-8) assay (Dojindo Laboratories). The CCK-8 assay is based on the conversion of a water-soluble tetrazolium salt, 2-(2-methoxy-4-nitrophenyl)-3-(4-nitrophenyl)-5-(2,4-disulfophenyl)-2H-tetrazolium, monosodium salt (WST-8), to a water-soluble formazan dye upon reduction by dehydrogenases in the presence of an electron carrier. The cells were incubated with 10% CCK-8 solution for 1-2 hour at 37°C. Then the absorbance of the culture medium was measured with a microplate reader at a test wavelength of 450 nm and a reference wavelength of 630 nm.

### Immunocytochemistry

The cells were washed twice with phosphate buffered saline (PBS), followed by 4% paraformaldehyde (PFA) for 15 minutes. After being further washed twice with PBS, they were incubated with PBS/0.1% Tween (10 min) and blocked with 3% BSA/PBS (1 hour at room temperature). The cells were incubated with primary antibodies against MAP2 (1:1000, Sigma Aldrich, M1406) at 4°C overnight. Subsequently, after being washed with PBS, they were incubated with secondary antibodies for 1 hour at room temperature. Finally, nuclei were counterstained with DAPI.

### ATP assay

ATP levels in cell lysates were measured with luciferase chemiluminescence-based ATP assay reagent for cells (Toyo Ink) in accordance with the manufacturer's protocol. Briefly, 100 µL of the lysis solution provided by the manufacturer was added to each well in 96-well plates. After incubating for 5 minutes at room temperature, luminescence of an aliquot of the solution was measured in a luminoneter.

### Western blots

Cells were collected and lysed in RIPA buffer (20 mM HEPES-KOH pH 7.4, 150 mM NaCl, 2 mM EDTA, 1% Nonidet-P40, 1% sodium deoxycholate) containing 10% 2-mercaptoethanol (Nacalai tesque) and 1% protease inhibitor (Nacalai tesque). Dissected brain samples were homogenized in the RIPA buffer with 2-mercaptomethanol and protease inhibitor. Samples were sonicated and centrifuged at 4°C, 15,000 rpm for 15 minutes, and the supernatants were collected. Proteins (15-20 µg per lane) were loaded, and were separated by 5-20% SDS-PAGE and transferred to polyvinylidene fluoride membranes (Millipore). The following primary antibodies were used: β-actin (1:5000, Sigma Aldrich, A5441), CHOP (1:1000, Cell Signaling Technology, L63F7), NeuN (1:2000, Merck Millipore, ABN78), VCP (1:500, ABGENT, AP6920b). HRP-labeled secondary antibodies (Santa Cruz Biotechnology) were used for visualization by enhanced chemiluminescence (Nacalai tesque).

### Mice

Adult male C57BL/6 (C57BL/6NJcl) and CB-17 (CB-17/lcr-+/+Jcl) mice (6-7 weeks old) were purchased from Shimizu Laboratory supplies and Clea Japan, respectively. Animals were allowed access to food and water ad libitum.

### Stroke surgery

In CB-17 mice, distal middle cerebral artery occlusion (distal MCAO) was performed as previously described with minor modifications (Kasahara Y, Ihara M, Nakagomi T, et al. A highly reproducible model of cerebral ischemia/reperfusion with extended survival in CB-17 mice. Neuroscience Research, 2013 Jul;76(3):163-8). In brief, general anesthesia was induced and maintained by inhalation of 4% and 1.5% isoflurane (Pfizer), respectively. Mice were placed in a lateral position, and a skin incision was made between the left eyeball and the left external auditory canal. The left salivary gland and a part of the temporalis muscle were resected to allow visualization of the middle cerebral artery (MCA) through the cranial bone. A burr hole was made in the cranial bone. Then, the MCA was isolated and transiently occluded with a monofilament nylon suture. After occlusion for 22 minutes, MCA blood flow was restored by the removal of the nylon suture. During surgery, rectal temperature was monitored and controlled at 36.0-37.2°C by a feedback-regulated heating pad.

In C57BL/6 mice, distal MCAO with hypoxia was performed as previously described with some modifications (Doyle KP, Fathali N, Siddiqui MR, et al. Distal hypoxic stroke: a new mouse model of stroke with high throughput, low variability and a quantifiable functional deficit. Journal of Neuroscience Methods, 2012 May 30;207(1):31-40). Mice were placed in a large chamber containing 10% oxygen and 90% nitrogen after the occlusion of the distal MCA by a nylon suture. After 30 minutes of hypoxia, mice were returned to normoxic condition with removal of the nylon suture. Sham surgery were identical to the stroke surgery, except for the occlusion of the distal MCA.

### Evaluation of stroke volume

Mice were deeply anesthetized and perfused transcardially with PBS and 4% PFA 24 hours after ischemia. The brains were extracted and post-fixed in 4% PFA for 48 hours. They were further cryoprotected in 20% sucrose until they sunk to the bottom of the vial. For Nissl staining, the brains were cut serially on a cryostat to 20 µm thick sections every 600 µm on slides (anterior-posterior +2.0, +1.4, +0.8, +0.2, -0.4, -1.0, and -1.6 mm relative to Bregma). The sections were incubated in cresyl violet solution for 15 minutes, and dehydrated in 70% methanol for 5 seconds. Afterwards, the slides were covered with mounting medium. The area of the ipsilateral hemisphere and the area of the infarct on each section were measured using NIH imageJ software. Measurements were multiplied by the distance between sections (600 µm) and then summed over the entire brain to yield volume measurements.

### Accelerating rotarod test

Twenty-four hours after ischemia, mice were placed on an accelerating rotarod apparatus, in which the speed was accelerated from 0 to 40 rpm over 4 minutes. The time for which mice can remain on the rotating cylinder was measured. Three trials were performed and the best latency to fall was used.

### Administration of KUS121

4-amino-3-[6-(4-fluoro-2-methylphenyl)pyridine-3-ylazo]naphthalene-1-sulfonic acid sodium salt prepared by the method described in WO2012/014994(Patent Literature 1), hereinafter referred to as KUS121 or KUS, was dissolved in 5% Cremophor EL (Sigma) in PBS to make a 20 µg/µL solution. In the focal ischemia stroke mouse model, the KUS121 solution was intravenously and intraperitoneally administered (100 mg/kg and 50 mg/kg of KUS121, respectively).

### Statistical analysis

All values are expressed as means ± SE unless stated otherwise. Statistical analysis was conducted using Dunnette's test (for cell viability tests) and Student's t test (for those other than cell viability tests). Differences with a probability value of p<0.05 were considered to be statistically significant.

### Results

### KUS121 protects primary cortical neurons under oxygen-glucose deprivation condition by preventing ATP depletion.

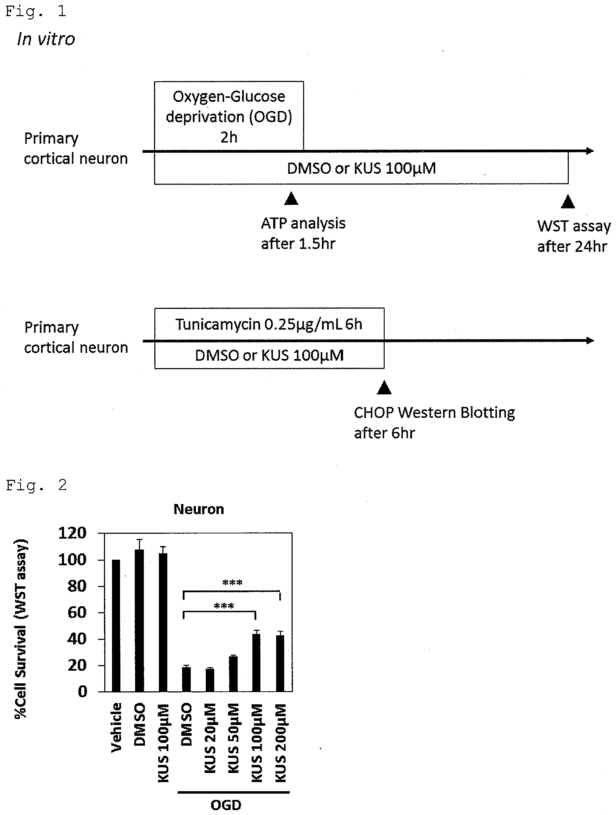

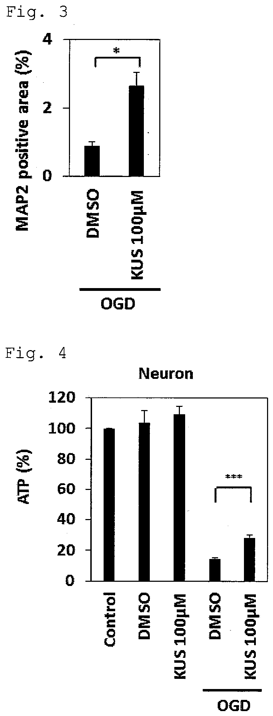

Fig. 1 shows procedures for *in vitro* experiments using primary cortical neurons. To assess protective effects of KUS121 on neurons in ischemic conditions, cell viability tests were performed after oxygen-glucose deprivation (OGD) in both the presence and the absence of KUS121 (Fig. 1, top). OGD was performed in rat cortical primary neuronal cultures for 2 hours with subsequent recovery at 21% O₂ and glucose-containing media for further 22 hours. Control neurons were kept at 21% O₂ in glucose-containing media for 24 hours. Exposure to OGD killed primary cortical neurons. However, the presence of 100 µM and 200 µM KUS121 throughout the experiment significantly improved neuronal viability after OGD (18.1±1.9% with vehicle (DMSO), 43.5±3.1% with 100 µM KUS, and 42.4±3.7% with 200 µM KUS; p<0.001) (Fig. 2). Furthermore, 100 µM KUS121 significantly increased the MAP2-positive area compared to vehicle (DMSO) (0.87±0.13% with vehicle (DMSO) and 2.64±0.41% with 100 µM KUS; p<0.05) (Fig. 3). DMSO or KUS121 without OGD had no effect on neuronal viability.

As KUS121 is reportedly able to prevent ATP depletion in pathological conditions, it was examined whether KUS121 showed similar effects in primary cortical neurons treated with OGD. Cellular ATP levels were measured by the luciferase-based assay after 1.5 hour OGD. The treatment with KUS increased ATP levels (14.3±1.1% with vehicle (DMSO) and 28.012.3% with 100 µM KUS; p<0.001) (Fig. 4). DMSO or KUS121 without OGD had no effect on the ATP levels. These data suggest that KUS121 protects primary cortical neurons under oxygen-glucose deprivation condition by preventing ATP depletion.

### KUS121 protects primary cortical neurons under ER stress-inducing condition.

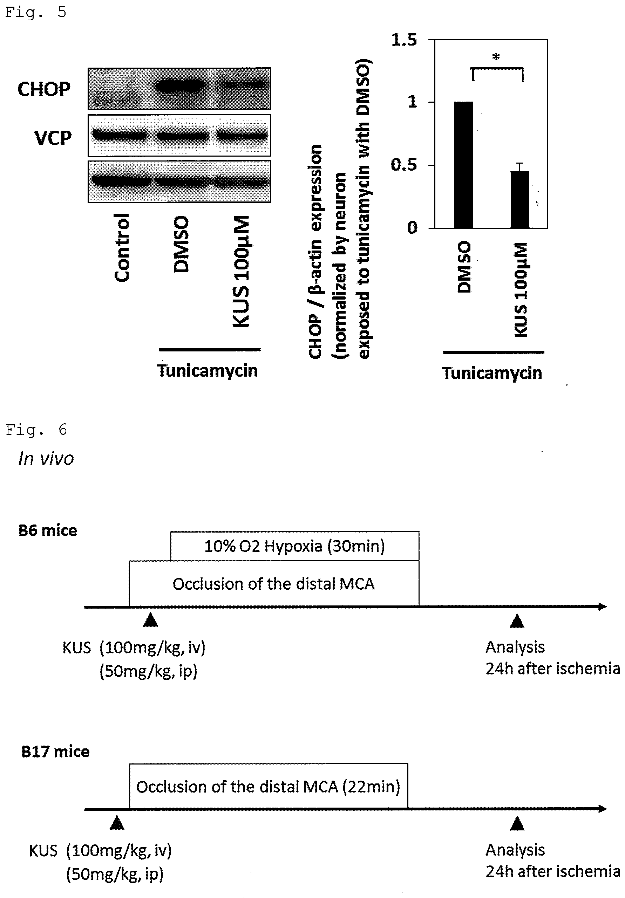

Because ER stress is triggered after ischemia, the protective effect of KUS121 was tested when primary cortical neurons were treated with tunicamycin (Fig. 1, bottom). Tunicamycin treatment is known to cause ER stress. C/EBP-homologous protein (CHOP) is a core mediator of ER stress-induced cell death, and is upregulated during ER stress. Cortical neurons were exposed to 0.25 µg/mL tunicamycin for 6 hours. KUS121 suppressed the expression of CHOP in primary cortical neurons treated with tunicamycin (Fig. 5).

### Treatment with KUS121 improves motor function and reduces brain infarction volume.

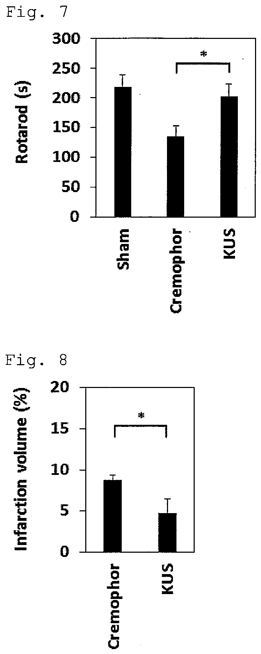

Fig. 6 shows procedures for *in vivo* experiments using mice. To evaluate the effect of KUS121 on cerebral ischemia, transient focal cerebral ischemia was induced in C57BL/6 mice. KUS121 was administered immediately after the occlusion of the distal portion of left middle cerebral artery (Fig. 6, top). Twenty-four hours after the occlusion, KUS121 significantly prolonged the rotarod retention time compared with vehicle (134.5±18.4 seconds with vehicle (5% Cremophor) and 201.0±21.8 seconds with KUS; p<0.05) (Fig. 7). In addition, infarction volumes were markedly reduced with KUS121 treatment (8.8±0.63% with vehicle (5% Cremophor) and 4.7±1.70% with KUS; p<0.05) (Fig. 8).

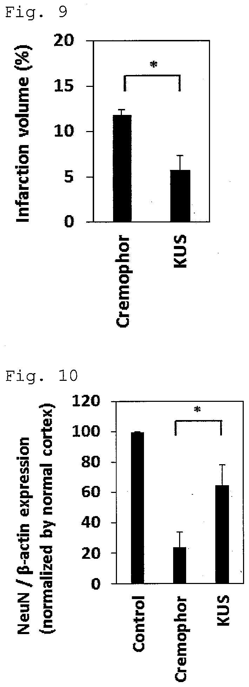

The neuroprotective effect was confirmed in CB-17 mice. Administration of KUS121 immediately before the ischemia reduced infarction volumes (11.8±0.60% with vehicle (5% Cremophor) and 5.74±1.64% with KUS; p<0.05) (Fig. 9). Furthermore, Western blotting of the cerebral cortex revealed that expression of NeuN, a neuron marker, was preserved with KUS121 (Fig. 10).

### INDUSTRIAL APPLICABILITY

The disclosure provides a method for treating cerebral infarction based on the mechanism that has not been used ever, and thus may be used in the field of medicine.

## Claims

1. A pharmaceutical composition for treating cerebral infarction, comprising a compound of formula (I): 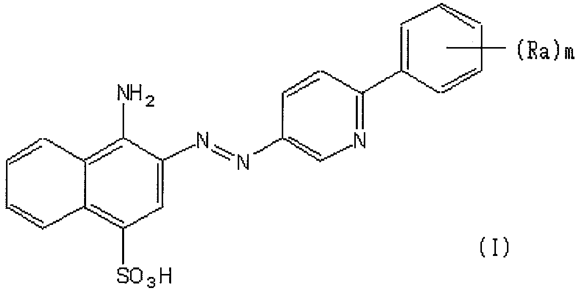 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt or solvate thereof.

2. The pharmaceutical composition according to claim 1, wherein each Ra radical is independently selected from the group consisting of halo, hydroxy, alkyl, halo-substituted alkyl, and alkoxy.

3. The pharmaceutical composition according to claim 1 or 2, wherein the compound of formula (I) is selected from the group consisting of
4-amino-3-(6-phenylpyridine-3-ylazo)naphthalene-1-sulfonic acid;
4-amino-3-(6-p-tolylpyridine-3-ylazo) naphthalene-1-sulfonic acid;
4-amino-3-(6-m-tolylpyridine-3-ylazo)naphthalene-1-sulfonic acid;
4-amino-3-(6-o-tolylpyridine-3-ylazo)naphthalene-1-sulfonic acid;
4-amino-3-(6-biphenyl-2-ylpyridine-3-ylazo)naphthalene-1-sulfonic acid;
3-[6-(2-acetylphenyl)pyridine-3-ylazo]-4-aminonaphthalene-1-sulfonic acid;
3-[6-(3-acetylphenyl)pyridine-3-ylazo]-4-aminonaphthalene-1-sulfonic acid;
3-[6-(4-acetylphenyl)pyridine-3-ylazo]-4-aminonaphthalenesulfonic acid;
4-amino-3-[6-(2,4-dichlorophenyl)pyridine-3-ylazo]naphthalene-1-sulfonic acid;
4-amino-3-[6-(2-trifluoromethylphenyl)pyridine-3-ylazo]naphthalene-1-sulfonic acid;
4-amino-3-[6-(4-trifluoromethylphenyl)pyridine-3-ylazo]naphthalene-1-sulfonic acid;
4-amino-3-[6-(2-chlorophenyl)pyridine-3-ylazo]naphthalene-1-sulfonic acid;
4-amino-3-[6-(3-chlorophenyl)pyridine-3-ylazo]naphthalene-1-sulfonic acid;
4-amino-3-[6-(4-chlorophenyl)pyridine-3-ylazo]naphthalene-1-sulfonic acid;
4-amino-3-[6-(2-methoxyphenyl)pyridine-3-ylazo]naphthalene-1-sulfonic acid;
4-amino-3-[6-(4-methoxyphenyl)pyridine-3-ylazo]naphthalene-1-sulfonic acid;
4-amino-3-[6-(2-isopropoxyphenyl)pyridine-3-ylazo]naphthalene-1-sulfonic acid;
4-amino-3-[6-(4-isopropoxyphenyl)pyridine-3-ylazo]naphthalene-1-sulfonic acid;
4-amino-3-[6-(2-phenoxyphenyl)pyridine-3-ylazo]naphthalene-1-sulfonic acid;
4-amino-3-[6-(3-methoxyphenyl)pyridine-3-ylazo]naphthalene-1-sulfonic acid;
4-amino-3-[6-(2,3-dimethylphenyl)pyridine-3-ylazo]naphthalene-1-sulfonic acid;
4-amino-3-[6-(2,5-dimethylphenyl)pyridine-3-ylazo]naphthalene-1-sulfonic acid;
4-amino-3-[6-(3,5-dimethylphenyl)pyridine-3-ylazo]naphthalene-1-sulfonic acid;
4-amino-3-[6-(3-trifluoromethylphenyl)pyridine-3-ylazo]naphthalene-1-sulfonic acid;
4-{4-[5-(1-amino-4-sulfonaphthalene-2-ylazo)pyridine-2-yl]phenyl}-4-oxobutyricacid;
4-amino-3-(6-biphenyl-3-ylpyridine-3-ylazo) naphthalene-1-sulfonic acid;
4-amino-3-[6-(3-cyanophenyl)pyridine-3-ylazo]naphthalene-1-sulfonic acid;
4-amino-3-[6-(4-cyanophenyl)pyridine-3-ylazo]naphthalene-l-sulfonic acid;
4-amino-3-[6-(3,5-bistrifluoromethylphenyl)pyridine-3-ylazo]naphthalenesulfonic acid;
4-amino-3-[6-(4-benzoylphenyl)pyridine-3-ylazo]naphthalene-1-sulfonic acid;
4-amino-3-[6-(2-propoxyphenyl)pyridine-3-ylazo]naphthalene-1-sulfonic acid;
4-amino-3-[6-(4-fluoro-2-methylphenyl)pyridine-3-ylazo]naphthalene-1-sulfonic acid;
4-amino-3-[6-(5-fluoro-2-propoxyphenyl)pyridine-3-ylazo]naphthalene-1-sulfonic acid;
4-amino-3-[6-(2-fluoro-6-propoxyphenyl)pyridine-3-ylazo]naphthalene-1-sulfonic acid;
4-amino-3-[6-(4-fluoro-2-propoxyphenyl)pyridine-3-ylazo]naphthalene-1-sulfonic acid;
4-amino-3-[6-(5-fluoro-2-methylphenyl)pyridine-3-ylazo]naphthalene-1-sulfonic acid;
4-amino-3-[6-(2-fluoro-5-methylphenyl)pyridine-3-ylazo]naphthalene-1-sulfonic acid;
4-amino-3-[6-(2-butoxyphenyl)pyridine-3-ylazo]naphthalene-1-sulfonic acid;
4-amino-3-[6-(2-hexyloxyphenyl)pyridine-3-ylazo]naphthalene-1-sulfonic acid;
4-amino-3-[6-(4-butylphenyl)pyridine-3-ylazo]naphthalene-1-sulfonic acid;
4-amino-3-[6-(2-hydroxyphenyl)pyridine-3-ylazo]naphthalene-1-sulfonic acid;
4-amino-3-{6-[2-(6-hydroxyhexyloxy)phenyl]pyridine-3-ylazo}naphthalene-1-sulfonic acid;
4-{2-[5-(1-amino-4-sulfonaphthalene-2-ylazo)pyridine-2-yl]phenoxy} butyric acid;
4-amino-3-{6-[2-(3-hydroxypropoxy)phenyl]pyridine-3-ylazo}naphthalene-1-sulfonic acid;
4-amino-3-[6-(2-isobutoxyphenyl)pyridine-3-ylazo]naphthalene-1-sulfonic acid;
4-amino-3-[6-(5-chloro-2-hydroxyphenyl)pyridine-3-ylazo]naphthalene-1-sulfonic acid;
4-amino-3-[6-(4-methylbiphenyl-2-yl)pyridine-3-ylazo]naphthalene-1-sulfonic acid;
4-amino-3-[6-(4'-chloro-4-methylbiphenyl-2-yl)pyridine-3-ylazo]naphthalene-1-sulfonic acid;
4-amino-3-[6-(4,3',5'-trimethylbiphenyl-2-yl)pyridine-3-ylazo]naphthalene-1-sulfonic acid;
4-amino-3-[6-(3'-chloro-4-methylbiphenyl-2-yl)pyridine-3-ylazo]naphthalene-1-sulfonic acid;
4-amino-3-[6-(2,6-dimethylphenyl)pyridine-3-ylazo]naphthalene-1-sulfonic acid;
4-amino-3-[6-(3-formyl-2-isopropoxy-5-methylphenyl)pyridine-3-ylazo]naphthalene-1-sulfonic acid; and
4-amino-3-[6-(3-formyl-2-butoxy-5-methylphenyl)pyridine-3-ylazo]naphthalene-1-sulfonic acid.

4. The pharmaceutical composition according to any one of claims 1 to 3, wherein the compound of formula (I) is 4-amino-3-[6-(4-fluoro-2-methylphenyl)pyridine-3-ylazo]naphthalene-1-sulfonic acid.

5. The pharmaceutical composition according to any one of claims 1 to 4, wherein the cerebral infarction is lacunar infarction, atherothrombotic cerebral infarction, or cardiogenic embolism.

6. The pharmaceutical composition according to any one of claims 1 to 5, wherein the composition is administered at the time of reperfusion after the cerebral infarction. 1

7. The pharmaceutical composition according to any one of claims 1 to 6, wherein the composition is administered within about 8 hours after the onset of the cerebral infarction.

8. The pharmaceutical composition according to any one of claims 1 to 7, wherein the composition is administered within about 4.5 hours after the onset of the cerebral infarction.

9. The pharmaceutical composition according to any one of claims 1 to 8, wherein the composition is used in combination with a thrombolytic agent.

10. The pharmaceutical composition according to claim 9, wherein the thrombolytic agent is tPA.

11. The pharmaceutical composition according to any one of claims 1 to 10, wherein the composition is intravenously administered.
